(19) Europäisches Patentamt
European Patent Office
Office européen des brevets

(11) Veröffentlichungsnummer: **0 003 128**
**B1**

(12) # EUROPÄISCHE PATENTSCHRIFT

(45) Veröffentlichungstag der Patentschrift: **07.01.81**

(51) Int. Cl.³: **C 07 C 41/06**, C 07 C 43/11, C 08 G 65/32

(21) Anmeldenummer: **79100061.5**

(22) Anmeldetag: **10.01.79**

(54) **Verfahren zur Herstellung von Dialkyläthern von Mono- und Polyäthylenglykolen.**

(30) Priorität: **17.01.78 DE 2801793**

(43) Veröffentlichungstag der Anmeldung: **25.07.79 Patentblatt 79/15**

(45) Bekanntmachung des Hinweises auf die Patenterteilung: **07.01.81 Patentblatt 81/1**

(84) Benannte Vertragsstaaten: **BE DE FR GB IT NL**

(56) Entgegenhaltungen: **DE-A-1 520 647**

(73) Patentinhaber: **BASF Aktiengesellschaft, Carl-Bosch-Strasse 38, D-6700 Ludwigshafen (DE)**

(72) Erfinder: **Platz, Rolf, Dr., Hansastrasse, D-6800 Mannheim 1 (DE)**
Erfinder: **Fuchs, Werner, Dr., Muenchbuschweg 30 a, D-6700 Ludwigshafen (DE)**
Erfinder: **Vodrazka, Wolfgang, Dr., Alzeyer Strasse 8, D-6713 Freinsheim (DE)**

**EP 0 003 128 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

# Verfahren zur Herstellung von Dialkyläthern von Mono- und Polyäthylenglykolen

Die vorliegende Erfindung betrifft ein verbessertes Verfahren zur Herstellung von Dialkyläthern von Mono- und Polyäthylenglykolen der allgemeinen Formel I

$$R^1\text{-O-(-}CH_2\text{-}CH_2\text{-O-)}_n\text{-}R^2 \qquad \text{I,}$$

in der $R^1$ einen Alkylrest mit 1 bis 4 C-Atomen, $R^2$ einen Alkylrest mit 3 bis 5 C-Atomen und n einen Wert von 1 bis 100 bedeutet.

Aus der DE-OS 2544569 ist es bekannt, diese Verbindungen durch Umsetzung eines Monoalkyläthers II mit einem Olefin III, welches dem Rest $R^2$ entspricht, in Gegenwart eines sauren Ionenaustauschers bei 2 bis 50 bar und 20 bis 150°C herzustellen, wie es am Beispiel des Propens als Olefin folgender Gleichung entspricht:

$$\underset{\text{II}}{R^1\text{-O-(-}CH_2\text{-}CH_2\text{-O)}_n\text{-H}} + \underset{\text{III}}{CH_2\text{=CH-}CH_3} \xrightleftharpoons{H^+\text{-Ionenaustauscher}}$$

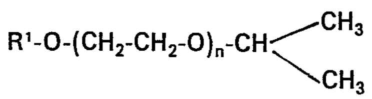

Da die anwendungstechnischen Eigenschaften von I durch die Anwesenheit von II merklich beeinträchtigt werden, nicht umgesetztes II indes praktisch nicht von I abgetrennt werden kann, muss man für einen möglichst vollständigen Umsatz von II sorgen.

Diesem Bestreben sind jedoch Grenzen gesetzt, da es sich, wie im Zusammenhang mit der Erfindung festgestellt wurde, um eine Gleichgewichtsreaktion handelt, wobei sich die Gleichgewichtslage mit steigender Temperatur zuungunsten der gewünschten Verfahrensprodukte I verschiebt.

Der Erfindung lag daher die Aufgabe zugrunde, den Umsatz der in Rede stehenden Reaktion zu erhöhen.

Es wurde gefunden, dass man den Umsatz bei dem Verfahren zur Herstellung von Dialkyläthern von Mono- und Polyoäthylenglykolen der allgemeinen Formel I

$$R^1\text{-O-(-}CH_2\text{-}CH_2\text{-O-)}_n\text{-}R^2 \qquad \text{I,}$$

in der $R^1$ einen Alkylrest mit 1 bis 4 C-Atomen, $R^2$ einen Alkylrest mit 3 bis 5 C-Atomen und n einen Wert von 1 bis 100 bedeutet, durch Umsetzung von Monoalkyläthern (II)

$$R^1\text{-O-(-}CH_2\text{-}CH_2\text{-O)}_n\text{-H} \qquad \text{II}$$

mit einem Olefin (III), welches dem Rest $R^2$ entspricht, in Gegenwart eines sauren Ionenaustauschers bei 1 bis 200 bar und 40 bis 150°C erhöhen kann, wenn man die Reaktion zunächst bei 100 bis 150°C beginnt und dann die Temperatur in mindestens zwei etwa gleichen Schritten auf 40 bis 80°C absenkt, wobei man jeweils dann auf die nächst niedere Stufe übergeht, wenn sich der Umsatz pro Zeiteinheit nicht mehr nennenswert ändert.

Theoretisch kann man das Verfahren in beliebig vielen Temperaturschritten ausführen, so dass die Temperaturabnahme stetig verlaufen würde. Aus praktischen Gründen empfiehlt es sich jedoch, die Temperatur stufenweise um jeweils 10 bis 20°C abzusenken.

Bei der bevorzugten Ausführungsform, beginnend mit 120°C und endend bei 180°C, entspricht dies vier Schritten der Temperatursenkung. Hierbei ist die Anfangstemperatur im allgemeinen diejenige Temperatur, welcher die Ionenaustauscher im Dauerbetrieb höchstens ausgesetzt werden dürfen, ohne thermisch geschädigt zu werden.

Die Massangabe, dass man das Umsetzungsgemisch jeweils solange auf einer bestimmten Temperaturstufe halten soll, bis sich der Umsatz pro Zeiteinheit nicht mehr nennenswert ändert, bedeutet praktisch, dass die Anfangstemperatur etwa 3 Stunden lang einzuhalten ist, und dass jede um 10°C tiefere Folgestufe etwa eine Stunde in Anspruch nimmt. Diese Zeiten verändern sich etwas mit der Katalysatormenge und dem Olefindruck, sind aber von der Art der Reaktanten praktisch nicht abhängig. Der Umsatz kann in einfacher Weise jeweils gaschromatographisch verfolgt werden. Die «Zeiteinheit» liegt für messtechnische Zwecke etwa zwischen 5 und 60 min, vorzugsweise etwa um 10 min.

Die Monoalkyläther II sind in bekannter Weise durch Umsetzung eines Alkohols $R^1$-OH mit Äthylenoxid in Gegenwart eines Katalysators wie Kaliumhydroxid erhältlich. Je nach den Mengenverhältnissen und den Reaktionsbedingungen erhält man hierbei Verbindungen II von unterschiedlichem Polymerisationsgrad n, und zwar in aller Regel Mischungen aus den Polymeren mit dem häufigsten Polymerisationsgrad $n_{max}$ und den benachbarten Polymerisationsgraden in der bekannten Gauss'schen Verteilung. Im Mittel entspricht dies einem mittleren Polymerisationsgrad $\bar{n}$, der sich von $n_{max}$ meistens nur geringfügig unterscheidet. Für die meisten anwendungstechnischen Zwecke werden Verbindungen I und damit Verbindungen II bevorzugt, in denen $\bar{n}$ zwischen 3 und 60 liegt. Innerhalb der angegebenen Definition hat die Art des Restes $R^1$ keinen nennenswerten Einfluss auf die Eigenschaften von I. Aus wirtschaftlichen und praktischen Gründen werden daher diejenigen Reste $R^1$ bevorzugt, die

sich von den billigen Alkoholen Methanol, Äthanol und Isopropanol ableiten.

Auch bei dem Rest $R^2$ kommt es in erster Linie darauf an, dass die freie -OH-Gruppe von II durch eine chemisch praktisch inerte Äthergruppierung als Endgruppe ersetzt wird. Man bevorzugt zur Verätherung daher das besonders reaktionsfähige und technisch gut verfügbare Propylen. Die angegebenen Reaktionszeiten gelten für das Propylen und die anderen n-Olefine, wogegen das noch reaktivere Isobuten deutlich mildere Reaktionsbedingungen beansprucht.

Die angegebenen Grenzen für den Druck des Olefins sind nicht als kritische Werte, sondern als Grenzen zu verstehen, innerhalb derer die Umsetzung am wirtschaftlichsten ist. Geringere Drucke verlangsamen die Reaktion zu sehr, höhere beschleunigen sie nicht mehr in der Weise, dass sich der grössere apparative und energetische Aufwand hierfür lohnen würde. Im allgemeinen genügt es, den Anfangsdruck des ersten Verfahrensschrittes bei der höchsten Temperatur über alle Temperaturstufen im wesentlichen konstant zu halten, jedoch kann man den Druck auch zur weiteren Erhöhung des Umsatzes bis zur Endstufe insgesamt um etwa 50 bar erhöhen.

Als stark saure Ionenaustauscher kommen insbesondere Sulfonsäuregruppen tragende vernetzte Styrol/Divinylbenzol-Copolymerisate in Betracht, wie sie unter den Bezeichnungen Lewatit ®SPC 118 BG, Amberlite® 200 und Amberlyst® 15 handelsüblich sind. Die Menge des Ionenaustauschers beträgt vorzugsweise 5 bis 20% der Ausgangsverbindung II. Vorteilhaft verwendet man den Ionenaustauscher in möglichst wasserfreier Form. Um Spuren anhaftenden Wassers zu entfernen, kann man ihn beispielsweise in Benzol aufschlämmen, worauf man das Benzol abdestilliert. Hierbei ist das Wasser im Azeotrop mit Benzol flüchtig.

Die Verfahrensprodukte I, von denen der Ionenaustauscher nach beendeter Reaktion nur noch abfiltriert zu werden braucht, sind wertvolle aprotische Flüssigkeiten, die sich vor allem für die Wäsche von Gasen eignen, indem sie saure Bestandteile wie Schwefelwasserstoff und Kohlendioxid zurückhalten.

Daneben eignen sich die Verfahrensprodukte als aprotische Lösungs- und Extraktionsmittel und Hydraulikflüssigkeiten. Der Umstand, dass sie bis zu 5 Prozentpunkte weniger Monoalkyläther enthalten als herkömmlich hergestellte Dialkyläther, verbessert ihre anwendungstechnischen Eigenschaften erheblich.

Beispiel

In einem Rührkessel von 2 $m^3$ Inhalt wurde ein Gemisch aus 1200 kg Oligoäthylenglykolmonomethyläther des mittleren Oligomerisationsgrades 5,5 und 120 kg des wasserfreien stark sauren Ionenaustauschers Lewatit SPC 118 BG unter einem Propylendruck von 35 bar und unter intensivem Rühren zunächst 3 Stunden lang auf 120°C gehalten, wonach die Temperatur unter Aufrechterhaltung des Druckes in Abständen von jeweils einer Stunde um jeweils 10°C bis auf 80°C gesenkt wurde. Nach der gaschromatographischen Analyse am Ende jeder Reaktionsstufe ergaben sich hierbei folgende Umsätze an Monoäther:

| Temperatur °C | Dauer h | Umsatz % |
|---|---|---|
| 120 | 3 | 90,0 |
| 110 | 1 | 93,3 |
| 100 | 1 | 94,8 |
| 90 | 1 | 95,2 |
| 80 | 1 | 96,5 |

Innerhalb der gesamten Reaktionszeit von 7 Stunden wurde bei einer Temperatur von durchgehend 120°C nur ein Umsatz von 93% erzielt, und bei durchgehend 80°C wurde nur ein Umsatz von knapp 10% erreicht.

**Patentanspruch**

Verfahren zur Herstellung von Dialkyläthern von Mono- und Polyäthylenglykolen der allgemeinen Formel I

$$R^1\text{-O-(-}CH_2\text{-}CH_2\text{-O-)}_n\text{-}R^2 \quad \text{I},$$

in der $R^1$ einen Alkylrest mit 1 bis 4 C-Atomen, $R^2$ einen Alkylrest mit 3 bis 5 C-Atomen und n einen Wert von 1 bis 100 bedeutet, durch Umsetzung von Monoalkyläthern II

$$R^1\text{-O-(-}CH_2\text{-}CH_2\text{-O)}_n\text{-H} \quad \text{II}$$

mit einem Olefin (III), welches dem Rest $R^2$ entspricht, in Gegenwart eines sauren Ionenaustauschers bei 1 bis 200 bar und 40 bis 150°C, dadurch gekennzeichnet, dass man die Reaktion zunächst bei 100 bis 150°C beginnt und dann die Temperatur in mindestens zwei etwa gleichen Schritten auf 40 bis 80°C absenkt, wobei man jeweils dann auf die nächst niedere Temperatur übergeht, wenn sich der Umsatz pro Zeiteinheit nicht mehr nennenswert ändert.

**Claim**

A process for the preparation of mono- and polyethylene glycol dialkyl ethers of the general formula I

$$R^1\text{-O-(-}CH_2\text{-}CH_2\text{-O-)}_n\text{-}R^2 \quad \text{I}$$

where $R^1$ is alkyl of 1 to 4 carbon atoms, $R^2$ is alkyl of 3 to 5 carbon atoms and n is from 1 to 100, by reacting monoalkyl ethers II

$$R^1\text{-O-(-}CH_2\text{-}CH_2\text{-O)}_n\text{-H} \quad \text{II}$$

with an olefin (III) which corresponds to the radical $R^2$, in the presence of an acid ion exchanger at from 1 to 200 bar and from 40 to 150°C, characterized in that the reaction is started at from 100 to 150°C and the temperature is then

lowered, in at least two approximately equal steps, to from 40 to 80°C, the temperature being dropped in each case to the next-lower level when the conversion per unit time no longer changes significantly.

**Revendication**

Procédé pour la préparation d'éthers dialcoyliques de mono- et de polyéthylène-glycols de formule générale I

$$R^1\text{-O-}(CH_2\text{-}CH_2\text{-O})_n\text{-}R^2 \qquad I$$

dans laquelle $R^1$ représente un radical alcoyle contenant 1 à 4 atomes de carbone, $R^2$ un radical alcoyle contenant 3 à 5 atomes de carbone en n a une valeur de 1 à 100, par réaction d'éthers monoalcoyliques II

$$R^1\text{-O-}(CH_2\text{-}CH_2\text{-O})_n\text{-H} \qquad II$$

avec une oléfine (III) qui correspond au radical $R^2$, en présence d'un échangeur d'ions acides, sous une pression de 1 à 200 bars et à une température de 40 à 150°C, caractérisé en ce qu'on fait tout d'abord débuter la réaction à une température de 100 à 150°C, puis on abaisse la température jusqu'à une valeur comprise entre 40 et 80°C en au moins deux échelons approximativement égaux, en ne passant chaque fois à l'échelon immédiatement inférieur que quand le degré de transformation par unité de temps ne varie plus de façon appréciable.